# EUROPÄISCHE PATENTSCHRIFT

(11) **EP 3 419 986 B1**
(45) Veröffentlichungstag und Bekanntmachung des Hinweises auf die Patenterteilung: **05.11.2025**
(21) Anmeldenummer: 17705130.7
(22) Anmeldetag: 15.02.2017
(51) Int. Cl.: C07H 15/06, C07H 1/00, A61Q 5/02, A61Q 13/00, A61K 8/60

(54) **RHAMNOLIPIDAMIDE ZUR HAARDUFTSTOFF-RETENTION**
RHAMNOLIPIDAMIDES FOR HAIR-PERFUME RETENTION
RHAMNOLIPIDAMIDES POUR LA RETENTION DES PARFUMS POUR LES CHEVEUX

(30) Priorität: 22.02.2016 EP 16156664
(43) Veröffentlichungstag der Anmeldung: 02.01.2019
(73) Patentinhaber: Evonik Operations GmbH, 45128 Essen (DE)
(72) Erfinder: LU, Xin, 45257 Essen (DE); NATTLAND, Sandra, 45359 Essen (DE); VAN LOGCHEM, Monica Desiree, 4762 PA Zevenbergen (NL); WENK, Hans Henning, 45470 Mülheim an der Ruhr (DE); CABIROL, Fabien, SG / 139009 (SG); DAHL, Verena, 51429 Bergisch Gladbach (DE); SCHEUERMANN, Ralph, 46236 Bottrop (DE); BRANDT, Kathrin Daniela, 40476 Düsseldorf (DE); KLEINEN, Jochen, 52525 Heinsberg (DE)
(74) Vertreter: Evonik Patent Association
(86) Internationale Anmeldenummer: PCT/EP2017/053349
(87) Internationale Veröffentlichungsnummer: WO 2017/144318

(56) Entgegenhaltungen:
- THIRKETTLE J ET AL: "SB-253514 AND ANALOGUES: NOVEL INHIBITORS OF LIPOPROTEIN-ASSOCIATED PHOSPHOLIPASE A2 PRODUCED BY PSEUDOMONAS FLUORESCENS DSM 11579. I. FERMENTATION OF PRODUCING STRAIN, ISOLATION AND BIOLOGICAL ACTIVITY", THE JOURNAL OF ANTIBIOTICS, NATURE PUBLISHING GROUP, GB, vol. 53, no. 7, 1 July 2000 (2000-07-01), pages 664 - 669, XP008064036, ISSN: 0021-8820
- YVONNE SCHMIDT ET AL: "Biosynthetic Origin of the Antibiotic Cyclocarbamate Brabantamide A (SB-253514) in Plant-Associated Pseudomonas .", CHEMBIOCHEM - A EUROPEAN JOURNAL OF CHEMICAL BIOLOGY., vol. 15, no. 2, 16 January 2014 (2014-01-16), DE, pages 259 - 266, XP055286251, ISSN: 1439-4227, DOI: 10.1002/cbic.201300527
- SHIDA MIAO ET AL: "Ethylation of Di-rhamnolipids: A Green Route to Produce Novel Sugar Fatty Acid Nonionic Surfactants", JOURNAL OF SURFACTANTS AND DETERGENTS, vol. 17, no. 6, 11 September 2014 (2014-09-11), DE, pages 1069 - 1080, XP055260723, ISSN: 1097-3958, DOI: 10.1007/s11743-014-1641-y
- WESTERDUIN P ET AL: "Synthesis of methyl 3-[3-(2-O-alpha-L-rhamnopyranosyl-alpha-L-rhamnopyranosyloxy)decanoyloxy]decanoate, a rhamnolipid from Pseudomonas aeruginosa", CARBOHYDRATE RESEARCH, PERGAMON, GB, vol. 180, no. 2, 15 September 1988 (1988-09-15), pages 195 - 205, XP026634128, ISSN: 0008-6215, [retrieved on 19880915], DOI: 10.1016/0008-6215(88)80077-6

## Beschreibung

### Gebiet der Erfindung

Gegenstand der Erfindung sind Derivate von Rhamnolipiden, Formulierungen enthaltend diese sowie deren Verwendung.

### Stand der Technik

Rhamnolipide sind Tenside, die mittels Fermentation hergestellt werden können. Sie setzen sich aus ein bis zwei Rhamnose-Einheiten und ein bis drei, meist β-Hydroxy-Fettsäuren zusammen. Die Fettsäuren können gesättigt oder ungesättigt sein. Die Variation in der Kettenlänge und Menge (Kongener) der Fettsäureanteile ist in einigen Veröffentlichungen beschrieben. (Howe et al., FEBS J. 2006; 273(22):5101-12; Abdel-Mawgoud et al., Appl Microbiol Biotechnol, 86, 2010; S. 1323-1336). Wenige kovalente Derivaten der Fettsäureanteile von Rhamnolipiden sind bekannt. Es sind hauptsächlich einige Rhamnolipidester in der Literatur beschrieben. Hirayama et al., FEBS Letters, Volume 139, Issue 1, 1982; Pages 81-85, beschreibt die Identifizierung von Rhamnolipidmethylestern in Flüssigkulturen von *Pseudomonas aeruginosa.* Miao et al., Journal of Surfactants and Detergents, 17 (6), 2014; 1069-1080 beschreibt die Synthese von di-Rhamnolipidethylester durch die Veresterung mit Ethanol.

Aufgabe der Erfindung war es, Substanzen bereitzustellen, die es einer Oberfläche, insbesondere der von Haaren, ermöglichen, einen Duft möglichst lange aufzuweisen.

### Beschreibung der Erfindung

Überraschenderweise wurde gefunden, dass die im Folgenden beschriebenen Rhamnolipidamide die der Erfindung gestellt Aufgabe zu lösen vermögen.

Gegenstand der vorliegenden Erfindung sind daher Rhamnolipidamide und deren Salze gemäß Anspruch 1.

Ein weiterer Gegenstand der Erfindung ist ein Verfahren zur Herstellung der erfindungsgemäßen Rhamnolipidamide sowie deren Verwendung.

Ein Vorteil der vorliegenden Erfindung ist, dass nach der Behandlungen mit den erfindungsgemäßen Formulierungen die Haare eine signifikant bessere Duftstoffretention aufweisen.

Ein weiterer Vorteil der vorliegenden Erfindung ist es, dass die erfindungsgemäßen Zusammensetzungen dem Haar einen schönen Glanz verleihen.

Noch ein Vorteil ist, dass die erfindungsgemäßen Zusammensetzungen in der Lage sind, die Kämmbarkeit und Formbarkeit von Haaren zu verbessern.

Noch ein Vorteil ist, dass das Herstellungsverfahren sehr mild und schonend ist, so dass die gewünschte Zuckerstruktur nicht zerstört wird, aber trotzdem eine Veresterung möglich ist.

Ein weiterer Vorteil ist, dass das Produkt sich hervorragend isolieren und aufarbeiten lässt.

Unter dem Begriff "Rhamnolipid" und "Rhamnolipidamid" im Zusammenhang mit der vorliegenden Erfindung werden auch immer deren korrespondierenden Salze mitumfasst.

Unter dem Begriff "Rhamnolipidamid" im Zusammenhang mit der vorliegenden Erfindung werden insbesondere Verbindungen der allgemeinen Formel (I), 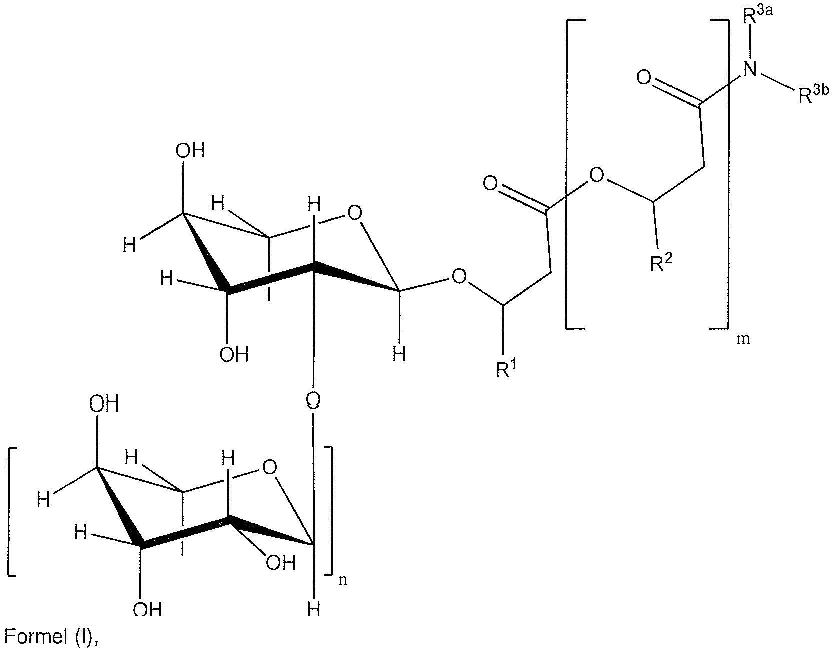 wobei
m = 2, 1 oder 0, insbesondere 1 oder 0,
n = 1 oder 0, insbesondere 1,
R¹ = organischer Rest mit 2 bis 24, bevorzugt 5 bis 13 Kohlenstoffatomen, ausgewählt aus gegebenenfalls verzweigter, gegebenenfalls hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12, R² = unabhängig voneinander gleicher oder verschiedener organischer Rest mit 2 bis 24, bevorzugt 5 bis 13 Kohlenstoffatomen, ausgewählt aus gegebenenfalls verzweigter, gegebenenfalls hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
R^{3a} = organischer Rest mit 2 bis 24, bevorzugt 3 bis 13, besonders bevorzugt 4 bis 8,
Kohlenstoffatomen, wobei R^{3a} ausgewählt ist aus der Gruppe der Alkylreste, welche gegebenenfalls Amingruppen aufweisen, insbesondere mit 4-8 Kohlenstoffatomen,
und R^{3b} = verstanden.

Unter dem Begriff "mono-Rhamnolipid" im Zusammenhang mit der vorliegenden Erfindung werden Verbindungen der allgemeinen Formel (I) mit -NR^{3a}R^{3b} = -OH oder deren Salze verstanden, bei denen n =0, verstanden.

Distinkte Rhamnolipide werden gemäß folgender Nomenklatur abgekürzt:
Unter "diRL-CXCY" werden di-Rhamnolipide der allgemeinen Formel (I) mit -NR^{3a}R^{3b} = -OH oder deren Salze verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Unter "monoRL-CXCY" werden mono-Rhamnolipide der allgemeinen Formel (I) mit -NR^{3a}R^{3b} = -OH oder deren Salze verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Die verwendete Nomenklatur unterscheidet somit nicht zwischen "CXCY" und "CYCX".

Für Rhamnolipide mit m=0 wird entsprechend monoRL-CX bzw. diRL-CX verwendet.

Ist einer der oben genannten Indices X und/oder Y mit ":Z" versehen, so bedeutet dies, dass der jeweilige Rest R¹ und/oder R² = ein unverzweigter, unsubstituierter Kohlenwasserstoffrest mit X-3 bzw. Y-3 Kohlenstoffatomen aufweisend Z Doppelbindungen darstellt.

Analoge Nomenklatur wird für Rhamnolipidamide in der Form di/monoRL-CXCY:Z-Amid eingesetzt.

Der "pH-Wert" im Zusammenhang mit der vorliegenden Erfindung ist definiert als der Wert, welcher für entsprechenden Stoff bei 25 °C nach fünf Minuten Rühren mit einer gemäß ISO 4319 (1977) kalibrierten pH-Elektrode gemessen wird.

Alle angegebenen Prozent (%) sind, wenn nicht anders angegeben, Massenprozent.

Erfindungsgemäße Rhamnolipidamide sind ausgewählt aus Verbindungen der allgemeinen Formel (I), wobei
m = 2, 1 oder 0, insbesondere 1 oder 0,
n = 1 oder 0, insbesondere 1,
R¹ = gegebenenfalls verzweigter, gegebenenfalls hydroxy-substituierter, gegebenenfalls ungesättigter, insbesondere gegebenenfalls einfach, zweifach oder dreifach ungesättigter, Alkylrest, mit 2 bis 24, bevorzugt 5 bis 13 Kohlenstoffatomen, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
R² = gegebenenfalls verzweigter, gegebenenfalls hydroxy-substituierter, gegebenenfalls ungesättigter, insbesondere gegebenenfalls einfach, zweifach oder dreifach ungesättigter, Alkylrest mit 2 bis 24, bevorzugt 5 bis 13 Kohlenstoffatomen, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
   dadurch gekennzeichnet, dass R^{3a} ausgewählt ist aus der Gruppe der Alkylreste mit 2 bis 24, bevorzugt 3 bis 13, besonders bevorzugt 4 bis 8, Kohlenstoffatomen, welche gegebenenfalls mindestens eine Amingruppe aufweisen, insbesondere mit 4 bis 8 Kohlenstoffatomen, und R^{3b} = H ist. In diesem Zusammenhang sind insbesondere Rhamnolipidamide bevorzugt ausgewählt aus diRLC10C10-Amiden, diC8C10-Amiden, diRLC10C12-Amiden, diRLC10C12:1-Amiden sowie monoRLC10C10-Amiden.

Die erfindungsgemäßen Rhamnolipidamide sind bevorzugt Mischungszusammensetzungen von Rhamnolipidamiden, die insbesondere dadurch gekennzeichnet sind, dass sie Mono- und Di-Rhamnolipidamide enthalten.

Je nach Anwendung kann es bevorzugt sein, dass die erfindungsgemäßen Mischungszusammensetzungen mehr Gewichtsprozente Mono-Rhamnolipidamide als Di-Rhamnolipidamide oder mehr Gewichtsprozente Di- Rhamnolipidamide als Mono-Rhamnolipidamide enthalten, wobei sich die Gewichtsprozente auf alle in der Mischungszusammensetzung enthaltenen Mono- und Di-Rhamnolipidamide beziehen.

So können beispielsweise die erfindungsgemäßen Mischungszusammensetzungen beispielsweise mehr als 60 Gew.-%, insbesondere mehr als 80 Gew.-%, oder gar mehr als 95 Gew.-%, Di-Rhamnolipidamide enthalten, oder aber auch beispielsweise mehr als 60 Gew.-%, insbesondere mehr als 80 Gew.-%, oder gar mehr als 95 Gew.-%, Mono-Rhamnolipidamide, wobei sich die Gewichtsprozente auf alle in der Mischungszusammensetzung enthaltenen Mono- und Di-Rhamnolipidamide beziehen.

Ein weiterer Gegenstand der vorliegenden Erfindung ist ein Verfahren zur Herstellung von den erfindungsgemäßen-Rhamnolipidamiden umfassend die Verfahrensschritte
A) Bereitstellen mindestens eines Rhamnolipides,
B) Umsetzen des Rhamnolipides mit mindestens einem Kopplungsreagens,
C) Umsetzen des durch Verfahrensschritt B) aktivierten Rhamnolipides mit einem Amin, und gegebenenfalls
D) Aufreinigen des Rhamnolipidamides.

Verfahrensschritt A) wird nach den allgemein bekannten Verfahren des Standes der Technik durchgeführt, insbesondere unter Einsatz von gentechnisch veränderten Mikroorganismen, die bevorzugt Rhamnolipidsynthesegene überexpremieren, wobei diese Gene bevorzugt ausgewählt sind aus rhIA, rhlB und rhIC. Entsprechende Anleitung findet der Fachmann in z.B. US2014296168 und WO2012013554.

Es ist erfindungsgemäß bevorzugt, dass in Verfahrensschritt B) als Kopplungsreagens mindestens eines ausgewählt aus der Gruppe umfassend, bevorzugt bestehend aus, Dicyclohexylcarbodiimid, Diisopropylcarbodiimid, 1-(3-Dimethylaminopropyl)-3-ethylcarbodiimidhydrochlorid, N-Cyclohexyl-N'-(2'-morpholinoethyl)carbodiimidmetho-p-toluolsulfonat, N-Benzyl-N'-3' dimethylaminopropylcarbodiimid-hydrochlorid, 1-Ethyl-3-(3-dimethylaminopropyl)carbodiimid N-Ethylcarbodiimid-hydrochlorid und Carbonyldiimidazol, insbesondere bevorzugt Dicyclohexylcarbodiimid und Diisopropylcarbodiimid, eingesetzt wird.

Ebenso ist es erfindungsgemäß bevorzugt, dass in Verfahrensschritt C) mindestens ein Katalysator ausgewählt aus der Gruppe umfassend, bevorzugt bestehend aus, N-Ethyldiisopropylamin, Trialkylamine, Pyridin, 4-Dimethylaminopyridin und Hydroxybenzotriazol, insbesondere Hydroxybenzotriazol, eingesetzt wird.

Erfindungsgemäß bevorzugte Verfahren führen bevorzugt zu den oben als erfindungsgemäß bevorzugt bezeichneten Rhamnolipidamiden.

So werden etwa bevorzugt in Verfahrensschritt A) bevorzugt Rhamnolipide ausgewählt aus diRLC10C10, diC8C10, diRLC10C12, diRLC10C12:1 sowie monoRLC10C10 oder Mischungen davon eingesetzt. Entsprechend bevorzugt ist der Einsatz von Aminen in Verfahrensschritt C) ausgewählt aus wobei
R⁷ = eine Alkylengruppe mit 1 bis 22, bevorzugt, 2 bis 18, insbesondere 3 bis 8 Kohlenstoffatomen,

Die erfindungsgemäßen Rhamnolipidamide lassen sich vorteilhaft in insbesondere kosmetischen Formulierungen einarbeiten.

Somit ist ein weiterer Gegenstand der vorliegenden Erfindung die
Verwendung der erfindungsgemäßen Rhamnolipidamide zur Herstellung von Formulierungen, insbesondere von kosmetischen Formulierungen,
sowie die Formulierungen, insbesondere kosmetische Formulierungen, welche die erfindungsgemäßen Rhamnolipidamide enthalten.

Die erfindungsgemäßen Formulierungen sind bevorzugt wässrige Formulierungen.

Unter dem Begriff "wässriges Formulierung" im Zusammenhang mit der vorliegenden Erfindung ist eine Formulierung zu verstehen, die mindestens 5 Gew.-% Wasser, bezogen auf die betrachtete Gesamtzusammensetzung enthält.

Es ist erfindungsgemäß bevorzugt, wenn die erfindungsgemäßen Formulierungen die erfindungsgemäßen Rhamnolipidamide in einer Menge von 0,05 Gew.-% bis 40 Gew.-%, bevorzugt von 0,2 Gew.-% bis 20 Gew.-%, besonders bevorzugt von 0,5 Gew.-% bis 12 Gew.-%, enthalten, wobei sich die Gewichtsprozente auf die Gesamtformulierung beziehen.

Bevorzugte erfindungsgemäße Formulierungen enthalten neben den erfindungsgemäßen Rhamnolipidamiden mindestens ein weiteres Tensid, wobei beispielsweise anionische, nichtionische, kationische und/oder amphotere Tenside eingesetzt werden können. Bevorzugt sind aus anwendungstechnischer Sicht Mischungen aus anionischen und nichtionischen Tensiden. Der Gesamttensidgehalt der wässrigen Formulierung beträgt vorzugsweise 5 bis 60 Gew.-% und besonders bevorzugt 15 bis 40 Gew.-%, bezogen auf die gesamte Formulierung.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C12-C14-Alkohole mit 3 EO, 4 EO oder 7 EO, C9-C11-Alkohol mit 7 EO, C13-C15- Alkohole mit 3 EO, 5 EO, 7 EO oder 8 EO, C12-C18-Alkohole mit 3 EO, 5 EO oder 7 EO und Mischungen aus diesen, wie Mischungen aus C12-C14-Alkohol mit 3 EO und C12-C18-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf. Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO(Propylenoxid)-Gruppen zusammen im Molekül enthalten, sind einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere.

Selbstverständlich sind auch gemischt alkoxylierte nichtionische Tenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Außerdem können als weitere nichtionische Tenside auch Alkylglykoside eingesetzt werden.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsaurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide; bei den Polyhydroxyfettsäureamiden handelt es sich um Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Als anionische Tenside werden beispielsweise solche vom Typ der Sulfonate und Sulfate eingesetzt. Als Tenside vom Sulfonat-Typ kommen dabei vorzugsweise C9-C13-Alkylbenzolsulfonate, Olefinsulfonate, d.h. Gemische aus Alken- und Hydroxyalkansulfonaten sowie Disulfonaten, wie man sie beispielsweise aus C12-C18-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C12-C18-Alkanen beispielsweise durch Sulfochlorierung oder Sulfoxidation mit anschließender Hydrolyse bzw. Neutralisation gewonnen werden. Ebenso sind auch die Ester von α -Sulfofettsäuren (Estersulfonate), zum Beispiel die α-sulfonierten Methylester der hydrierten Kokos-, Palmkern- oder Talgfettsäuren geeignet.

Weitere geeignete anionische Tenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C12-C18-Fettalkohole, beispielsweise aus Kokosfettalkohol, Talgfettalkohol, Lauryl-, Myristyl-, Cetyl- oder Stearylalkohol oder der C10-C20-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C12-C16-Alkylsulfate und C12-C18-Alkylsulfate sowie C14-C18-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN^{®} erhalten werden können, sind geeignete anionische Tenside. Auch die Schwefelsäuremonoester der mit 1 bis 6 Mol Ethylenoxid ethoxylierten geradkettigen oder verzweigten C7-C20-Alkohole, wie 2-Methyl-verzweigte C9-C11-Alkohole mit im Durchschnitt 3,5 Mol Ethylenoxid (EO) oder C12-C18-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete anionische Tenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C8-C18-Fettalkoholreste oder Mischungen aus diesen. Insbesondere bevorzugte Sulfosuccinate enthalten einen Fettalkoholrest, der sich von ethoxylierten Fettalkoholen ableitet. Dabei sind wiederum Sulfosuccinate, deren Fettalkohol-Reste sich von ethoxylierten Fettalkoholen mit enger Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Als amhpotere Tenside können erfindungsgemäß solche oberflächenaktiven Verbindungen eingesetzt werden, die im Molekül mindestens eine quartäre Ammoniumgruppe und mindestens eine -COO⁻- oder -SO₃⁻ -Gruppe tragen. Besonders bevorzugte amphotere Tenside sind in diesem Zusammenhang Betain-Tenside wie Alkyl- oder Alkylamidopropylbetaine. Insbesondere sind hier Betaine wie die N-Alkyl-N,N-dimethylammonium-glycinate, z. B. das Kokosalkyldimethylammoniumglycinat, N-Acyl-aminopropyl-N,N-dimethylammoniumglycinate, z. B. das Kokosacylaminopropyldimethylammoniumglycinat, das C12-C18-Alkyl-dimethyl-acetobetain, das Kokosamidopropyl-dimethyl-acetobetain, 2-Alkyl-3-carboxymethyl-3-hydroxyethyl-imidazoline und Sulfobetaine mit jeweils 8 bis 18 C-Atomen in der Alkyl- oder Acylgruppe sowie das Kokosacylaminoethylhydroxyethylcarboxymethylglycinat bevorzugt. Ein besonders bevorzugtes zwitterionisches Tensid ist das unter der INCI-Bezeichnung Cocamidopropyl Betaine bekannte N,N-Dimethyl-N-(lauroylamidopropyl)ammoniumacetobetain.

Weitere geeignete amphotere Tenside bildet die Gruppe der Amphoacetate und Amphodiacetate, insbesondere beispielsweise Kokos- oder Laurylamphoacetate oder -diacetate, die Gruppe der Amphopropionate und Amphodipropionate sowie die Gruppe der aminosäurebasierten Tenside wie Acylglutamate, insbesondere Disodium Cocoyl Glutamate und Sodium Cocoyl Glutamate, Acylglycinate, insbesondere Cocoyl Glycinate, und Acylsarcosinate, insbesondere Ammonium Lauroyl Sarcosinate und Sodium Cocoyl Sarcosinate.

Insbesondere bevorzugt enthalten die erfindungsgemäßen Formulierungen einen Duftstoff.

Die erfindungsgemäßen Formulierungen können des Weiteren mindestens eine zusätzliche Komponente enthalten, ausgewählt aus der Gruppe der
Emollients,
Emulgatoren,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Die erfindungsgemäßen Rhamnolipidamide sowie die erfindungsgemäßen Formulierungen enthaltend die erfindungsgemäßen Rhamnolipidamide lassen sich vorteilhaft zur Reinigung von Oberflächen verwenden. Bei dieser Form der erfindungsgemäßen Verwendung ist die Oberfläche bevorzugt die Oberfläche eines Lebewesens, insbesondere eines Menschen, wobei solche Oberflächen besonders bevorzugt ausgewählt sind aus Haut und Haar, insbesondere Haar.

Ein weiterer Gegenstand der vorliegenden Erfindung ist die Verwendung der erfindungsgemäßen Rhamnolipidamide und/oder der erfindungsgemäßen Formulierungen zur Duftstoffretention, insbesondere auf Haaren.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Herstellung von di-Rhamnolipiden

Eine Fermentation mit einem rekombinanten Stamm *Pseudomonas putida* KT2440S *pBBR1MCS2-Plac-rhlABC-T-Ptac-rhlC-T* wurde durchgeführt. Die Konstruktion des Stamms wird beschrieben in der US2014296168. Die Vorkultur im Schüttelkolben wurde wie in WO2012013554 beschrieben durchgeführt. Für die Hauptkultur kam ebenfalls ein Mineralmedium (M9) zum Einsatz. Die Fermentation erfolgt in einem glukoselimitierten Fed-Batch - Verfahren in einem 2 Liter Fermenter. Die Glukose-Zufütterung wird anhand des Gelöstsauerstoffsignals reguliert. Der Sauerstoffpartialdruck der Fermentationsbrühe wurde bei 20 % Sättigung über die Rührerdrehzahl reguliert. Der pH-Wert wird über eine pH Elektrode und Zugabe von 2M Schwefelsäure bzw. einer 20 Gew.-% Ammoniaklösung auf 7 reguliert. Um das Überschäumen der Fermentationsbrühe zu verhindern, wurde der Entschäumer DOW Corning 1500 bei Bedarf zu dosiert. Die Fermentation wurde über 4 Tage bis zu einer Biotrockenmasse von 15 g/l geführt. Die Rhamnolipidkonzentration wurde über HPLC ermittelt und betrug 9,8 g/l. Nach Abtrennen der Zellen mittels Zentrifugation bei 10.000 g wurde die Fermentationsbrühe durch Zugabe konzentrierter H₂SO₄ auf einen pH-Wert von 3,1 eingestellt. Durch erneute Zentrifugation bei wurde ein pastöses Feststoff-Konzentrat mit einem RL-Anteil von 45 Gew.-% und mit einer Viskosität von > 10.000 mPas gewonnen. Unter ständigem Rühren wurde eine 50 gew.-%ige wässrige KOH Lösung zur pastösen Suspension des aufkonzentrierten Rhamnolipidpräzipitats gegeben und ein pH-Wert von 6 eingestellt. Hierbei kam es zur Verflüssigung der pastösen Masse, die mit einem starken Viskositätsabfall einherging. Aus der Suspension wurde eine klare Lösung. Durch Zugabe von Wasser wurde die Lösung auf einen Aktivgehalt von 35 Gew.-% eingestellt. Die Rhamnolipid-Reinheit betrug > 90 Gew.-% bezogen auf die Trockenmasse.

Mittels HPLC nachgewiesene Rhamnolipidspezies waren:

| | | |
|---|---|---|
| RL gesamt [%] (HPLC) | | **91** |
| | diRL-C8C10 | 13,9 |
| | monoRL-C8C10 | 0,51 |
| | diRL-C10C10 | 61,4 |
| | monoRL -C10C10 | 1,4 |
| | diRL-C10C12:1 | 5,9 |
| | diRL-C10C12 | 5,5 |
| | other RL | 2,2 |

### Beispiel 2: Herstellung von mono-Rhamnolipiden

Die wie oben beschrieben hergestellte, 35 Gew.-% Rhamnolipidlösung wurde durch Zugabe von Wasser auf 1 % verdünnt. Zwei Liter dieser Lösung wurden auf 50 °C erwärmt. Unter leichtem Rühren wurden 200 Units einer thermostabilen Rhamnosidase (ThermoActiveTM Rhamnosidase A, Prokazyme) zugegeben und die Reaktion über Nacht durchgeführt. Nach 20 h wurde eine Probe der Lösung mittels HPLC analysiert. Das di-Rhamnolipid war vollständig zu mono-Rhamnolipid und Rhamnose umgesetzt worden. Anschließend wurde das Enzym bei 80 °C eine Stunde inaktiviert.

Dann wurde der gesamte Ansatz gefriergetrocknet. Das gefriergetrocknete Produkt wurde durch Wasserzugabe auf einen mono-Rhamnolipid Aktivgehalt von 35 Gew.-% eingestellt.

### Beispiel 3a: Synthese von di-Rhamnolipid-Hexylamid

Zur Aktivierung der Säurefunktion werden 25g di-Rhamnolipid (40 mmol) mit 6,25 ml Diisopropylcarbodiimid (40mmol) bei 55°C in THF gelöst. Ist eine Säurezahl von < 2 erreicht, werden 4,86 g Hexylamin (48mmol) zugegeben, sowie 1Gew% 4-Dimethylamidopyridin zur Katalyse. Das entstehende Reaktionswasser fördert die Bildung von N,N'-Diisopropylharnstoff als Nebenkomponente. Nach einer Reaktionzeit von 5 Stunden wird das Reaktionsgemisch am Rotationsversampfer getrocknet (45°C, < 300mbar), eine Aufreinigung erfolgt durch Ausschütteln mit Ethylacetat (1): Wasser (1) (2 x jeweils 20 ml) um den entstanden Harnstoff abzutrennen. Die Ethylacetatphase wird eingedampft (Rotationsverdampfer, 45°C, < 300mbar) und das Rhamnolipidhexylamid verbleibt als Feststoff.

Weitere Aufreinigung des Produkts kann mittels Säulenchromatographie erfolgen. Dazu dient Silica 60 gel (SIGMA Aldrich) als stationäre Phase und Ethylacetat (99) : Wasser (1) mit 1% Essigsäure als mobile Phase. Aus einer 5%igen Lösung des Rohprodukts werden Hexylamin Reste, polare Neben- oder eventuelle Spaltprodukte entfernt. Zur sorgfältigen Auftrennung umfasst eine Fraktion 10ml bei einer Tropfgeschwindigkeit von 15 ml / min und einem Gesamtvolumen von 200 ml Ausgangslösung.

### Beispiel 4: Beschreibung der anwendungstechnischen Effekte und der Formulierung

Um den Einfluss der genannten Strukturen auf die Retention von Duftstoffen auf Haaren zu bewerten wurde ein olfaktorischer Anwendungstest durchgeführt.

Die für den Geruchstest verwendeten Haarbündel der Fa. Kerling wurden zunächst mit einem einfachen Shampoo bestehend aus einer wässrigen Lösung von 12% Sodium Laureth Sulfate, die mit Natriumchlorid auf eine Viskosität von ca. 2500 mPa s eingestellt ist, gemäß folgender Vorgehensweise vorgewaschen:
Die Haarbündel wurden unter fließendem, warmem Wasser benetzt. Das überschüssige Wasser wurde leicht von Hand ausgedrückt, dann wurde das Shampoo aufgebracht und sanft im Haar eingearbeitet (1 ml/Haarsträhne (2 g)). Nach einer Verweilzeit von 1 min wurde das Haar für 1 min gespült. Die vorgewaschenen, feuchten Haarbündel wurden anschließend mit folgenden Shampoo-Formulierungen gewaschen.

### Formulierungen 2-3 und 7

| | Formulierung 2 | Formulierung 3 | Formulierung 7 (erfindungsgemäß) |
|---|---|---|---|
| Sorbitan Sesquicaprylate | 0,2 % | 0,2 % | 0,2 % |
| Sodium Laureth Sulfate | 7,5 % | 7,5 % | 7,5 % |
| Geraniol | 0,3 % | - | 0,3 % |
| Quaternium-80 | 1,0 % | 1,0 % | 1,0 % |
| Aqua / Water | ad 100 % | ad 100 % | ad 100 % |
| Cocamidopropyl Betaine | 3,5 % | 3,5 % | 3,5 % |
| PEG-18 Glyceryl Oleate/Cocoate | 2,5 % | 2,5 % | 2,5 % |
| Glycol Distearate | 1,0 % | 1,0 % | 1,0 % |
| | - | - | |
| Rhamnolipidamide gemäß Beispiel 3a | - | - | 3,0 % |

### Formulierungen 45-6 sowie 8:

| | Formulierung 5 | Formulierung 6 | Formulierung 8 (erfindungsgemäß) |
|---|---|---|---|
| Sorbitan Sesquicaprylate | 0,2 % | 0,2 % | 0,2 % |
| Sodium Laureth Sulfate | 6,5 % | 6,5 % | 6,5 % |
| Citronellol | 0,2 % | - | 0,2 % |
| Quaternium-80 | 1,0 % | 1,0 % | 1,0 % |
| Aqua / Water | ad 100 % | ad 100 % | ad 100 % |
| Cocamidopropyl Betaine | 3,0 % | 3,0 % | 3,0 % |
| PEG-18 Glyceryl Oleate/Cocoate | 2,5 % | 2,5 % | 2,5 % |
| Glycol Distearate | 1,0 % | 1,0 % | 1,0 % |
| | - | - | |
| Rhamnolipidamide gemäß Beispiel 3a | - | - | 2,0 % |

Nach einer 24-stündigen Trocknung im Klimaraum bei 25°C und 50% rel. Luftfeuchte wurde der olfaktorische Eindruck der Haare von einem geschulten Panel bestehend aus 15 Panelisten gemäß folgendem Schema bewertet:
0 kein Duftstoff-spezifischer Geruch wahrnehmbar
1 Duftstoff-spezifischer Geruch gerade noch wahrnehmbar
2 ausgeprägter Duftstoff-spezifischer Geruch wahrnehmbar

Die Ergebnisse der olfaktorischen Beurteilung der wie oben beschrieben durchgeführten Behandlung der Haarbündel mit der erfindungsgemäßen Formulierung 7 und den Ergebnissen aus den Vergleichsformulierungen 2 und 5 sowie den Kontrollformulierungen 3 und 6 (Placebo ohne Duftstoff) werden in der folgenden Tabelle gegenübergestellt:

### Ergebnisse aus dem Geruchspanel:

| Panelnote | | Formulierung 2 | Formulierung 3 | Formulierung 7 (erfindungsgemäß) |
|---|---|---|---|---|
| nach 24 h | | 1,8 | 0,0 | 1,8 |
| nach 48 h | | 0,8 | 0,0 | 1,7 |
| nach 96 h | | 0,2 | 0,1 | 1,5 |
| nach 168 h | | 0,1 | 0,0 | 1,4 |

| Panelnote | | Formulierung 5 | Formulierung 6 | Formulierung 8 (erfindungsgemäß) |
|---|---|---|---|---|
| nach 24 h | | 1,6 | 0,0 | 1,5 |
| nach 48 h | | 0,7 | 0,1 | 1,5 |
| nach 96 h | | 0,4 | 0,0 | 1,4 |
| nach 168 h | | 0,0 | 0,0 | 1,2 |

Überraschenderweise zeigen die Ergebnisse des Geruchspaneltests, dass die mit den erfindungsgemäßen Formulierungen 7 und 8 behandelten Haare eine signifikant bessere Duftstoffretention aufweisen als die Vergleichsformulierungen 2 und 5 und die Kontrollformulierungen 3 und 6.

## Patentansprüche

1. Rhamnolipidamid gemäß der allgemeinen Formel (I), 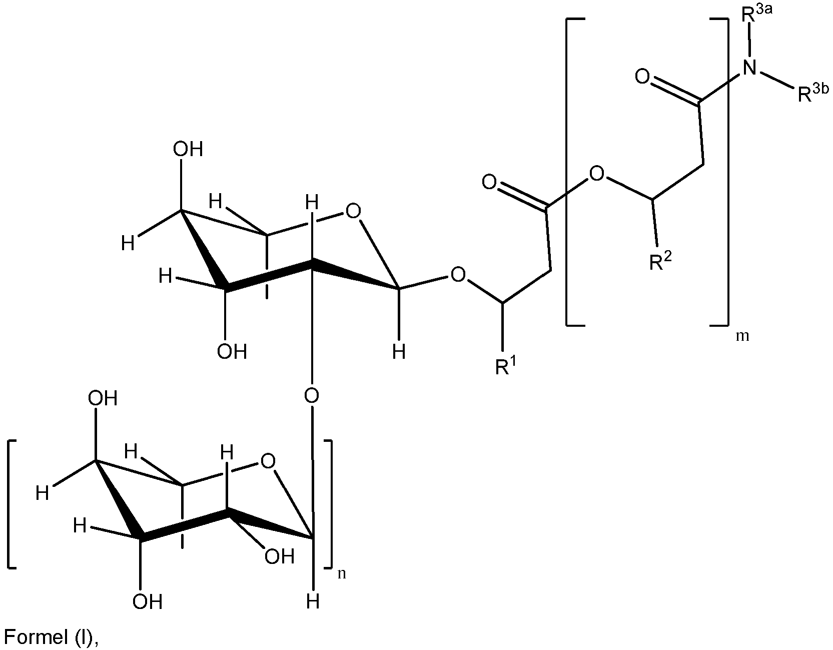 wobei
m = 2, 1 oder 0, insbesondere 1 oder 0,
n = 1 oder 0, insbesondere 1,
R¹ = organischer Rest mit 2 bis 24, bevorzugt 5 bis 13 Kohlenstoffatomen, ausgewählt aus gegebenenfalls verzweigter, gegebenenfalls hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
R² = unabhängig voneinander gleicher oder verschiedener organischer Rest mit 2 bis 24, bevorzugt 5 bis 13 Kohlenstoffatomen, ausgewählt aus gegebenenfalls verzweigter, gegebenenfalls hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
R^{3a} = organischer Rest mit 2 bis 24, bevorzugt 3 bis 13, besonders bevorzugt 4 bis 8, Kohlenstoffatomen, und
R^{3b} = organischer Rest mit 2 bis 24, bevorzugt 3 bis 13, besonders bevorzugt 4 bis 8, Kohlenstoffatomen oder H, bevorzugt H,
**dadurch gekennzeichnet, dass**
R^{3a} ausgewählt ist aus der Gruppe der Alkylreste, welche gegebenenfalls Amingruppen aufweisen, insbesondere mit 4 bis 8 Kohlenstoffatomen, und
R^{3b} = H.

2. Verfahren zur Herstellung von Rhamnolipidamiden nach Anspruch 1 umfassend die Verfahrensschritte
A) Bereitstellen mindestens eines Rhamnolipides,
B) Umsetzen des Rhamnolipides mit mindestens einem Kopplungsreagens,
C) Umsetzen des durch Verfahrensschritt B) aktivierten Rhamnolipides mit einem Amin, und gegebenenfalls
D) Aufreinigen des Rhamnolipidamides.

3. Verfahren nach Anspruch 2, **dadurch gekennzeichnet, dass** in Verfahrensschritt B) als Kopplungsreagens mindestens eines ausgewählt aus der Gruppe umfassend, bevorzugt bestehend aus, Dicyclohexylcarbodiimid, Diisopropylcarbodiimid, 1-(3-Dimethylaminopropyl)-3-ethylcarbodiimidhydrochlorid, N-Cyclohexyl-N'-(2'-morpholinoethyl)carbodiimidmetho-p-toluolsulfonat, N-Benzyl-N'-3' dimethylaminopropylcarbodiimid-hydrochlorid, 1-Ethyl-3-(3-dimethylaminopropyl)carbodiimid N-Ethylcarbodiimid-hydrochlorid und Carbonyldiimidazol, insbesondere bevorzugt Dicyclohexylcarbodiimid und Diisopropylcarbodiimid, eingesetzt wird.

4. Verfahren nach Anspruch 2 oder 3, **dadurch gekennzeichnet, dass** in Verfahrensschritt C) mindestens ein Katalysator ausgewählt aus der Gruppe umfassend, bevorzugt bestehend aus, N-Ethyldiisopropylamin, Trialkylamine, Pyridin, 4-Dimethylaminopyridin und Hydroxybenzotriazol, insbesondere Hydroxybenzotriazol, eingesetzt wird..

5. Rhamnolipidamid nach Anspruch 1 erhältlich nach einem Verfahren nach mindestens einem der Ansprüche 2 bis 4.

6. Formulierung, insbesondere eine kosmetische, enthaltend mindestens ein Rhamnolipidamid nach mindestens einem der Ansprüche 1 oder 5.

7. Verwendung eines Rhamnolipidamides nach mindestens einem der Ansprüche 1 oder 5 oder einer Formulierung nach Anspruch 6 zur Duftstoffretention, insbesondere auf Haaren.

## Claims

1. Rhamnolipid amide of the general formula (I), 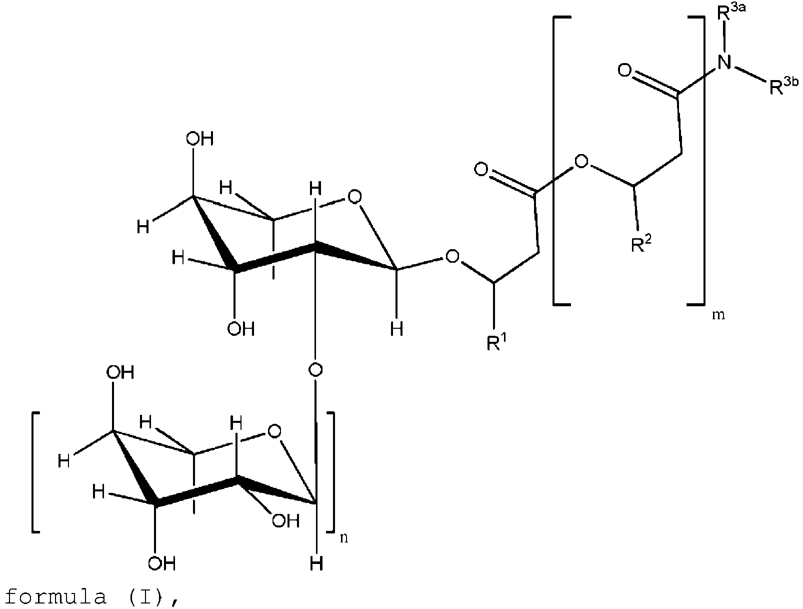 where
m = 2, 1 or 0, in particular 1 or 0,
n = 1 or 0, in particular 1,
R¹ = organic radical having 2 to 24, preferably 5 to 13, carbon atoms, selected from optionally branched, optionally hydroxy-substituted, optionally unsaturated, in particular optionally mono-, bi- or tri-unsaturated, alkyl radical, preferably those selected from the group consisting of pentenyl, heptenyl, nonenyl, undecenyl and tridecenyl and (CH₂)ₒ-CH₃ where o = 1 to 23, preferably 4 to 12,
R² = independently of one another identical or different organic radical having 2 to 24, preferably 5 to 13, carbon atoms, selected from optionally branched, optionally hydroxy-substituted, optionally unsaturated, in particular optionally mono-, bi- or tri-unsaturated, alkyl radical, preferably those selected from the group consisting of pentenyl, heptenyl, nonenyl, undecenyl and tridecenyl and (CH₂)ₒ-CH₃ where o = 1 to 23, preferably 4 to 12,
R^{3a} = organic radical having 2 to 24, preferably 3 to 13, particularly preferably 4 to 8, carbon atoms, and
R^{3b} = organic radical having 2 to 24, preferably 3 to 13, particularly preferably 4 to 8, carbon atoms or H, preferably H,
**characterized in that**
R^{3a} is selected from the group of the alkyl radicals which optionally have amine groups, in particular having 4 to 8 carbon atoms, and
R^{3b} = H.

2. Process for the preparation of rhamnolipid amides according to Claim 1 comprising the process steps
A) provision of at least one rhamnolipid,
B) reaction of the rhamnolipid with at least one coupling reagent,
C) reaction of the rhamnolipid activated by process step B) with an amine, and optionally
D) purification of the rhamnolipid amide.

3. Process according to Claim 2, **characterized in that** in process step B) the coupling reagent used is at least one selected from the group comprising, preferably consi sting of, dicyclohexylcarbodiimide, diisopropylcarbodiim ide, 1-(3-dimethylaminopropyl)-3-ethylcarbodiimide hydro chloride, N-cyclohexyl-N'-(2'-morpholinoethyl)carbodiim ide metho-p-toluenesulfonate, N-benzyl-N'-3'-dimethylami nopropylcarbodiimide hydrochloride, 1-ethyl-3-(3-dimethy laminopropyl)carbodiimide, N-ethylcarbodiimide hydrochlo ride and carbonyldiimidazole, especially preferably dicy clohexylcarbodiimide and diisopropylcarbodiimide.

4. Process according to Claim 2 or 3, **characterized in that** in process step C) at least one catalyst selected from the group comprising, preferably consisting of, N-ethyldiisopropylamine, trialkylamines, pyridine, 4-dimethylaminopyridine and hydroxybenzotriazole, in particular hydroxybenzotriazole, is used.

5. Rhamnolipid amide according to Claim 1 obtainable by a process according to at least one of Claims 2 to 4.

6. Formulation, in particular a cosmetic one, comprising at least one rhamnolipid amide according to at least one of Claims 1 and 5.

7. Use of a rhamnolipid amide according to at least one of Claims 1 and 5 or of a formulation according to Claim 6 for fragrance retention, in particular on hair.

## Revendications

1. Rhamnolipidamide selon la formule générale (I), 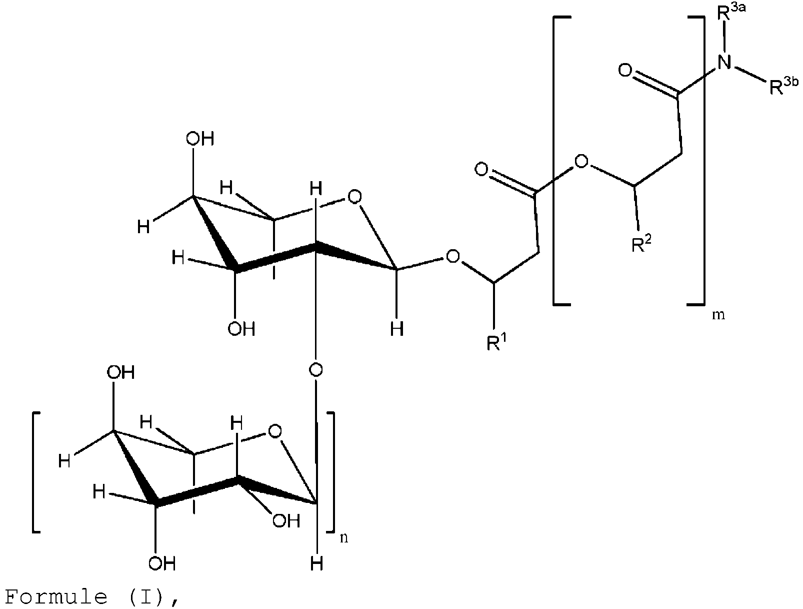 dans laquelle
m = 2, 1 ou 0, en particulier 1 ou 0,
n = 1 ou 0, en particulier 1,
R¹ = radical organique ayant de 2 à 24, de préférence 5 à 13 atomes de carbone, choisi parmi un radical alkyle éventuellement ramifié, éventuellement substitué par hydroxy, éventuellement insaturé, en particulier éventuellement une fois, deux fois ou trois fois insaturé, de préférence un tel radical choisi dans le groupe constitué par les groupes pentényle, heptényle, nonényle, undécényle et tridécényle et (CH₂)ₒ-CH₃ où o = 1 à 23, de préférence 4 à 12,
R² = radical organique identique ou différent chaque fois indépendamment, ayant de 2 à 24, de préférence 5 à 13 atomes de carbone, choisi parmi un radical alkyle éventuellement ramifié, éventuellement substitué par hydroxy, éventuellement insaturé, en particulier éventuellement une fois, deux fois ou trois fois insaturé, de préférence un tel radical choisi dans le groupe constitué par les groupes pentényle, heptényle, nonényle, undécényle et tridécényle et (CH₂)ₒ-CH₃ où o = 1 à 23, de préférence 4 à 12,
R^{3a} = radical organique ayant de 2 à 24, de préférence 3 à 13, de façon particulièrement préférée 4 à 8, atomes de carbone, et
R^{3b} = radical organique ayant de 2 à 24, de préférence 3 à 13, de façon particulièrement préférée 4 à 8, atomes de carbone, ou H, de préférence H,
**caractérisé en ce que**
R^{3a} est choisi dans le groupe des radicaux alkyle qui comportent éventuellement des groupes amino, en particulier ayant de 4 à 8 atomes de carbone, et
R^{3b} = H.

2. Procédé pour la préparation de rhamnolipidamides selon la revendication 1, comprenant les étapes de procédé
A) mise à disposition d'au moins un rhamnolipide,
B) mise en réaction du rhamnolipide avec au moins un réactif de couplage,
C) mise en réaction du rhamnolipide, activé par l'étape
B) du procédé, avec une amine, et éventuellement
D) purification du rhamnolipidamide.

3. Procédé selon la revendication 2, **caractérisé en ce que** dans l'étape B) du procédé est utilisé en tant que réactif de couplage au moins un choisi dans le groupe comprenant, de préférence consistant en, le dicyclohexylcarbodiimide, le diisopropylcarbodiimide, le chlorhydrate de 1-(3-diméthylaminopropyl)-3-éthylcarbodiimide, le métho-p-toluènesulfonate de N-cyclohexyl-N'-(2'-morpholinoéthyl)carbodiimide, le chlorhydrate de N-benzyl-N'-3' diméthylaminopropylcarbodiimide, le chlorhydrate de 1-éthyl-3-(3-diméthylaminopropyl)carbodiimide, le chlorhydrate de N-éthylcarbodiimide et le carbonyldiimidazole, de façon particulièrement préférée le dicyclohexylcarbodiimide et le diisopropylcarbodiimide.

4. Procédé selon la revendication 2 ou 3, **caractérisé en ce que** dans l'étape C) du procédé est utilisé au moins un catalyseur choisi dans le groupe comprenant, de préférence consistant en, la N-éthyldiisopropylamine, les trialkylamines, la pyridine, la 4-diméthylaminopyridine et l'hydroxybenzotriazole, en particulier l'hydroxybenzotriazole.

5. Rhamnolipidamide selon la revendication 1, pouvant être obtenu conformément à un procédé selon au moins l'une quelconque des revendications 2 à 4.

6. Formulation, en particulier une formulation cosmétique, contenant au moins un rhamnolipidamide selon au moins l'une quelconque des revendications 1 ou 5.

7. Utilisation d'un rhamnolipidamide selon au moins l'une quelconque des revendications 1 ou 5 ou d'une formulation selon la revendication 6, pour la rétention de parfum, en particulier sur des cheveux.
